Europäisches Patentamt

European Patent Office

Office européen des brevets

(19)

(11) Publication number: **0 046 335**
**A2**

(12) **EUROPEAN PATENT APPLICATION**

(21) Application number: **81303247.1**

(22) Date of filing: **15.07.81**

(51) Int. Cl.³: **C 12 P 7/62**
// C12N1/06

(30) Priority: **18.08.80 GB 8026882**

(43) Date of publication of application: **24.02.82**
**Bulletin 82/8**

(84) Designated Contracting States: **BE DE FR GB IT NL**

(71) Applicant: **IMPERIAL CHEMICAL INDUSTRIES PLC,**
**Imperial Chemical House Millbank, London SW1P 3JF**
**(GB)**

(72) Inventor: **Holmes, Paul Arthur, Middlesbrough Cleveland,**
**middlesbrough Cleveland (GB)**
Inventor: **Jones, Eric, 94 Quarry Road, Tarporley**
**Cheshire (GB)**

(74) Representative: **Gratwick, Christopher et al, Imperial**
**Chemical Industries PLC Legal Department: Patents**
**Thames House North Millbank, London SW1P 4QG (GB)**

(54) **Extraction of poly(beta-hydroxybutyric acid).**

(57) Poly(β-hydroxybutyric acid), PHB, extraction from a
bacterial cell suspension by heating to above 100°C
under pressure, releasing the pressure, and separating
PHB granules from the cell debris.

EP 0 046 335 A2

1

Extraction of poly(β-hydroxybutyric acid)

This invention relates to the extraction of poly(β-hydro-oxybutyric acid) hereinafter referred to as PHB.

PHB is a thermoplastic polyester that is useful as a plastics material. It is accumulated by many bacteria as an energy reserve material in the form of granules within the bacterial cells.

While bacterial cells containing PHB can be used as such as a moulding material, for example as described in USP 3,107,172, it is generally desirable to separate the PHB from the remainder of the bacterial cell material.

Methods that have been proposed to effect this separation include breakage of the cells by methods such as treatment with acetone, followed by extraction of the PHB from the broken cells by treatment with a solvent in which PHB is soluble. Examples of such processes are described in USP's 3,036,959 and 3,044,942 in which the solvents employed are pyridine or mixture of methylene chloride and ethanol. Other extraction solvents for PHB in the form in which it is produced in the bacterial cells include cyclic carbonates such as 1,2-propylene carbonate (see USP 4,101,533); chloroform (see USP 3,275,610); and 1,2-dichloroethane (as disclosed in our European Patent Application 15123).

USP 3,275610 discloses other methods of cell breakage viz. ultrasonic vibration, grinding, French pressing, freezing/thawing cycles and lysozyme treatment, while, as described in the aforementioned European Patent Application, spray or flash drying of the suspension of cells, as produced by culturing the microorganism in an aqueous medium on a suitable carbon and energy source, can also cause sufficient cell breakage to enable the PHB to be extracted from the cells.

One disadvantage of these processes is that it is necessary to separate the PHB-containing solution from the cell debris. Because of the small size of the bacterial cells, and hence of the fragments resulting therefrom, techniques such as filtration have heretofore presented difficulties. This is particularly true where the PHB-containing solution is relatively viscous as in the case where chloroform is utilised as the solvent. As described in the aforesaid European Patent Application, in some cases the PHB can be extracted by a wet process wherein the aqueous bacterial cell suspension, after a suitable cell breakage step, is contacted with a suitable solvent for PHB and then the solvent and aqueous phases are separated. However, separation of the phases may be slow and incomplete.

We have now devised a process for the separation of PHB whereby such difficulties are avoided.

The process of the present invention stems from the discovery that, by treatment of the cell suspension under certain conditions, the bacterial cells can be broken to such an extent that the resultant suspension contains cell debris and discrete PHB granules. By application of suitable techniques e.g. flotation and/or subjecting the suspension to an electric field, separation of the cell debris from the PHB granules can be achieved.

Accordingly we provide a process for the extraction of PHB from an aqueous suspension of bacterial cells containing PHB comprising heating said suspension to a temperature exceeding 100°C under sufficient pressure to maintain said suspension in the liquid state and then releasing the pressure on said heated suspension, whereby a suspension containing cell debris and discrete granules of PHB is obtained, and then separating the granules of PHB from the cell debris.

The suspension of cell debris and discrete PHB granules

may be produced by pumping the bacterial cell suspension under pressure through a heated zone provided with an orifice through which the heated suspension is expelled into a region of lower pressure. The temperature of the zone and the flow rate of the suspension should be such that the suspension is heated to above $100^{\circ}C$, preferably to above $150^{\circ}C$, but the pressure applied should be sufficient to maintain the suspension in the liquid state. Heating may alternatively be effected by injecting steam under pressure into the suspension. On release of the pressure, e.g. on emergence from the orifice, the liquid inside the bacterial cells tends to vaporise causing the cells to disintegrate.

Both the cell debris and the PHB granules have a surface charge as a result of polar groups thereon and so, by suitable selection of pH conditions, application of an electric field to the suspension of cell debris and PHB granules will cause migration of the PHB granules relative to the cell debris, thus effec ing separation thereof.

The separation of the cell debris and PHB granules may also be effected by gravimetric techniques since the cell debris and PHB granules have differing densities; generally the cell debris will be lighter than the PHB granules.

Before or after separation from the cell debris, PHB granules may be caused to flocculate, if desired, in order to assist separation thereof from the aqueous medium, by addition of a solvent, such as methanol or acetone, in which PHB is insoluble, but which dissolves the lipids associated with the PHB granules.

The resultant PHB granules, whether or not subjected to such a lipid removal step, can be separated from the aqueous medium by filtration or centrifugation. Alternatively, after separation of the cell debris from the PHB granules, the PHB granules may be separated from the aqueous medium by spray or flash drying. The resultant dried PHB can be subjected to a lipid extraction step, e.g. with acetone or methanol, if desired.

The invention is illustrated by the following example.

200 ml of cold aqueous suspension containing 10 g l$^{-1}$ of cells of <u>Azotobacter</u> <u>beijerinckii</u> in which the cells contained about 50% by weight of PHB was charged to a first stirred autoclave. 220 ml of water was charged to a second stirred autoclave and heated under nitrogen pressure to 400$^{\circ}$C. The first autoclave was pressurised with nitrogen to a pressure exceeding that in the second autoclave and then the contents of the first autoclave were forced into the second autoclave by the nitrogen pressure excess. The combined contents of the second autoclave were mixed vigorously for two minutes. The temperature of the combined contents of the second autoclave was approximately 220$^{\circ}$C but the applied pressure was sufficient to maintain the contents in the liquid state.

The contents of the second autoclave was then forced by the nitrogen pressure through a fine jet into a collection tank containing about 20 litres of cold water.

The resulting thin bacterial suspension was examined by high resolution light microscopy which revealed massive cell breakage: discrete granules of PHB having diameters in the range 0.2 to 0.5 μm were clearly visible.

To a portion of the suspension an equal volume of acetone was added. The acetone dissolved the lipid coating of the PHB granules and caused the resultant hydrophobic granules to coagulate. The coagulated granules could be separated from the aqueous medium containing the cell debris by contrifugation. Washing the separated coagulated granules removed residual impurities.

1. A process for the extraction of poly(β-hydroxybutyric acid), PHB, from an aqueous suspension of bacterial cells containing PHB comprising heating said suspension to a temperature exceeding 100°C under sufficient pressure to maintain said suspension in the liquid state, and then releasing the pressure on said heated suspension whereby a suspension containing cell debris and discrete granules of PHB is obtained, and then separating the granules of PHB from the cell debris.

2. A process according to claim 1 wherein the pressure is released by expelling the heated suspension under pressure through an orifice into a region of lower pressure.

3. A process according to claim 1 or claim 2 wherein the suspension is heated to a temperature exceeding 150°C.

4. A process according to any one of claims 1 to 3 wherein the suspension is heated by injecting steam under pressure into the suspension.

5. A process according to any one of claims 1 to 3 wherein the suspension is heated by pumping the suspension under pressure through a heated zone.

6. A process according to any one of claims 1 to 5 wherein the PHB granules are separated from the cell debris by application of an electrical field to the suspension after release of the pressure thereon.

7. A process according to any one of claims 1 to 5 wherein the PHB granules are separated from the cell debris gravimetrically.

8. A process according to any one of claims 1 to 7 wherein the PHB granules are separated from the cell debris to give an aqueous suspension of PHB granules free of cell debris, and the PHB granules are then separated from the cell-debris-free aqueous medium.

9. A process according to any one of claims 1 to 8 wherein, before or after separation of the PHB granules from the cell debris, lipids associated with the PHB granules are dissolved by addition of a solvent, in which the lipids are soluble but in which PHB is insoluble, to the aqueous suspension containing the PHB granules.

10. A process according to claim 8 wherein the PHB is separated from the cell-debris-free aqueous medium by spray or flash drying.

11.      A process according to claim 10 in which lipids associated with the PHB are removed by extraction of the dried PHB with a solvent in which the lipids are soluble but in which PHB is insoluble.